# FASCICULE DE BREVET EUROPEEN

(11) **EP 1 068 854 B1**
(45) Date de publication et mention de la délivrance du brevet: **18.08.2004**
(21) Numéro de dépôt: 00401661.4
(22) Date de dépôt: 13.06.2000
(51) Int. Cl.: A61K 7/02, A61K 7/027, A61K 7/031, A61K 7/48

(54) **Composition sous forme solide comprenant une huile et un composé gélifiant particulier, procédé de traitement cosmétique et utilisation dudit composé**
Ein spezifisches Geliermittel und ein Öl enthaltende Zusammensetzung in fester Form, Verfahren zur kosmetischen Behandlung und Verwendung dieser Mittel
Solid form composition comprising an oil and a specific gelling agent, process for cosmetic treatment and use of this compound

(30) Priorité: 15.07.1999 FR 9909178
(43) Date de publication de la demande: 17.01.2001
(73) Titulaire: L'OREAL, 75008 Paris (FR)
(72) Inventeur: Livoreil, Aude, 75006 Paris (FR); Mougin, Nathalie, 75011 Paris (FR)
(74) Mandataire: Dodin, Catherine

(56) Documents cités:
- PATENT ABSTRACTS OF JAPAN vol. 1998, no. 13, 30 novembre 1998 (1998-11-30) & JP 10 212213 A (POLA), 11 août 1998 (1998-08-11)
- H. STETTER ET AL : "Cyclisierungsreaktionen ausgehend von 1.3.5-Triamino-cyclohexan" CHEMISCHE BERICHTE., vol. 103, 1970, pages 200-204, XP002151568 VERLAG CHEMIE GMBH. WEINHEIM., DE ISSN: 0009-2940
- K. HANABUSA ET AL: "Small molecular gelling agents to harden organic liquids: Trialkyl cis-1,3,5-cyclohexanetricarboxamides" CHEMISTRY LETTERS., 1997, pages 191-192, XP002151569 CHEMICAL SOCIETY OF JAPAN. TOKYO., JP ISSN: 0366-7022

## Description

La présente invention a trait à une composition solide cosmétique, telle qu'une composition de soin, de traitement et/ou de maquillage de la peau, y compris du cuir chevelu, et/ou des lèvres des êtres humains, ladite composition comprenant une phase grasse liquide épaissie, et se présentant notamment sous forme d'un stick ou bâton de maquillage comme un rouge à lèvres, dont l'application peut conduire à un dépôt brillant et non-migrant.

Dans les compositions notamment cosmétiques et dermatologiques, il est courant d'utiliser une phase grasse liquide structurée, c'est-à-dire épaissie ou gélifiée, pour obtenir la consistance souhaitée. L'épaississement des huiles (ou des phases liquides à température ambiante) permet en particulier de faciliter la prise du produit hors de son conditionnement sans perte significative, de limiter la diffusion du produit à la zone locale de traitement, de répartir le produit de façon régulière sur la zone locale de traitement ou bien encore de pouvoir utiliser le produit dans des quantités suffisantes pour obtenir l'effet cosmétique ou dermatologique recherché Ceci est notamment le cas dans les compositions solides comme les déodorants, les baumes et les rouges à lèvres, les produits anti-cerne et les fonds de teint coulés. Cet épaississement est notamment primordial pour les compositions de soin, d'hygiène ou de maquillage comme les rouges à lèvres qui doivent bien se répartir de façon homogène sur la surface locale à traiter ainsi que pour les compositions capillaires qui doivent s'étaler et se répartir de façon régulière le long des fibres kératiniques et ne pas ruisseler sur le front, la nuque, le visage ou dans les yeux.
Pour remédier à ces problèmes, on a habituellement recourt à des cires ou des charges. Malheureusement, ces cires et/ou charges ont tendance à matifier la composition et à la rendre opaque, ce qui n'est pas toujours souhaitable en particulier pour un rouge à lèvres. En effet, les femmes sont toujours à la recherche d'un rouge à lèvres sous forme d'un bâton permettant l'obtention d'un film brillant; par ailleurs, certaines compositions telles que les baumes à lèvres ou les onguents, peuvent se présenter sous forme de sticks translucides, voire transparents.
Il est également connu d'épaissir les huiles avec des épaississants polymériques. Malheureusement, les épaississants d'huiles connus doivent être utilisés en grande quantité pour obtenir un gel de viscosité élevée, par exemple supérieure à 1,3 Pa.s. Or, une trop grande quantité d'épaississant peut conférer à la composition des propriétés cosmétiques inadéquates, notamment un toucher collant et un manque de glissant, ces inconvénients pouvant être très gênants, voire rédhibitoires.
La structuration de la phase grasse liquide permet en particulier de limiter son exsudation des compositions solides et, en plus, de limiter, après dépôt sur la peau ou les lèvres, la migration de cette phase dans les rides et ridules, ce qui est particulièrement recherché pour un rouge à lèvres. En effet, une migration importante de la phase grasse liquide, chargée de matières colorantes, conduit à un effet inesthétique autour des lèvres, accentuant particulièrement les rides et les ridules. Cette migration est souvent citée par les femmes comme un défaut majeur des rouges à lèvres classiques.

JP-A-10 212 213 décrit des compositions cosmétiques solides telles que des sticks de rouges à lèvres comprenant un dérivé de cyclohexane tricarboxamide en une quantité de 20 % un poids et des cires en une quantité de 30 % en poids.

La présente invention a pour but de proposer l'obtention d'une composition cosmétique, se présentant sous forme solide, et comprenant peu, voire pas de cires, tout en étant susceptible de conserver de bonnes propriétés cosmétiques, et notamment une certaine translucidité.

L'invention a donc pour objet une composition cosmétique ou dermatologique, se présentant sous forme solide, comprenant au moins une huile et au moins un composé défini par la formule 1 telle que décrite ci-après, ladite composition comprenant moins de environ 5% en poids de cire, par rapport au poids total de la composition.
Notamment, la composition peut se présenter sous la forme d'un stick anhydre translucide voire transparent. Elle trouve une application particulière comme composition "sans transfert" ou "non migrante" éventuellement colorée.

Un autre objet de l'invention est un procédé de traitement cosmétique d'un support choisi parmi la peau du visage ou du corps, les muqueuses et les fibres kératiniques, comprenant l'application sur ledit support d'une composition telle que définie ci-dessus.

Un autre objet de l'invention est l'utilisation, dans une composition cosmétique ou dermatologique se présentant sous forme solide, comprenant au moins une huile et comprenant moins de environ 5% en poids de cire par rapport au poids total de la composition, d'une quantité suffisante d'au moins un composé de formule I, pour structurer/gélifier ladite composition.

On a en effet constaté que l'utilisation des composés de formule I permet de structurer les phases grasses liquides, ou phases huileuses, voire de les gélifier complètement, et ainsi d'obtenir des compositions cosmétiques stables sous forme gélifiée solide, pouvant être exemptes de cires. Ceci est vrai même pour une très faible teneur en composé de formule I.
La composition selon l'invention présente de bonnes propriétés cosmétiques : elle n'est pas collante lors de l'application et est glissante et facile à appliquer. Elle permet l'obtention d'un film homogène et uniforme, couvrant et confortable à porter.
De plus, la composition peut avantageusement être claire, transparente ou translucide.
On entend par là la définition classique donnée dans le dictionnaire. Ainsi, une composition translucide laisse passer la lumière sans permettre toutefois de distinguer nettement les contours des objets. Une composition transparente se laisse aisément traverser par la lumière et permet de distinguer nettement les objets à travers son épaisseur.
D'une manière générale, une composition transparente aura une valeur de transmittance maximum de la lumière, quelque soit la longueur d'onde comprise entre 400 et 800 nm, à travers un échantillon de 1 cm d'épaisseur, d'au moins 35%, de préférence d'au moins 50% (voir EP291334).
Une composition translucide aura, quant à elle, une valeur de transmittance maximum de la lumière comprise entre 2 et 35%.
La transmittance peut être mesurée en plaçant un échantillon de 1 cm d'épaisseur dans le rayon lumineux d'un spectrophotomètre travaillant dans les longueurs d'onde du spectre lumineux.

Par ailleurs, les composés de formule I peuvent avantageusement être utilisés pour préparer des compositions "sans transfert" notamment colorées, pour lesquelles la migration du film coloré dans les rides et ridules, notamment autour des lèvres ou des yeux/ est très limitée. Ces compositions présentent également l'avantage de ne pas, ou peu, se déposer sur certains supports avec lesquels elles sont mises en contact, tels que, par exemple, un verre, un vêtement ou la peau.

La composition selon l'invention comprend donc au moins un composé correspondant à la formule I : 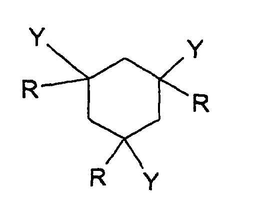 dans laquelle :
* R représente, indépendamment les uns des autres, un atome d'hydrogène ou une chaîne hydrocarbonée saturée, linéaire ou ramifiée, ayant 1 à 6 atomes de carbone, notamment 1 à 4 atomes de carbone;
* Y représente un groupement choisi parmi les groupements suivants : -CO-S-R'; -CO-NHR'; -NH-COR' et -S-COR'; dans lesquels R' représente, indépendamment les uns des autres :
   - un atome d'hydrogène,
   - un groupement aryle,
   - un groupement aralkyle, c'est-à-dire aryle substitué par une chaîne hydrocarbonée saturée, linéaire ou ramifiée, comprenant 1 à 22 atomes de carbone, notamment 10-18 atomes de carbone; ou
   - une chaîne hydrocarbonée saturée, linéaire, ramifiée ou cyclique, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy.

De préférence, R représente un atome d'hydrogène.
De préférence, Y représente un groupement -CO-NHR'ou -NH-COR'.
De préférence, R' représente un groupement aryle; un groupement aralkyle dans lequel la chaîne alkyle, linéaire ou ramifiée, comprend 12-16 atomes de carbone; ou une chaîne alkyle linéaire ou ramifiée en C12-18.

Encore plus préférentiellement, Y représente un groupement -CO-NHR' dans lequel R' représente un groupement aryle substitué par une chaîne alkyle linéaire ou ramifiée, en C12-C16; ou R' représente une chaîne alkyle linéaire ou ramifiée, en C12-C18, non substituée.

Les trois substituants représentés par Y peuvent être, dans le composé de formule I, en conformation cis-cis, cis-trans ou trans-trans, les uns par rapport aux autres. Notamment, au moins un de ces substituants peut être placé en position équatoriale sur le cycle cyclohexane; de préférence, tous les substituants Y sont placés en position équatoriale.

Parmi les composés susceptibles d'être employés dans le cadre de l'invention, on peut citer :
- le cis-1,3,5-tris(dodecylaminocarbonyl)cyclohexane,
- le cis-1,3,5-tris(octadecylaminocarbonyl)cyclohexane,
- le cis-1,3,5-tris[N-(3,7-diméthyloctyl)-aminocarbonyl]cyclohexane,
- le trans-1,3,5-triméthyl-1,3,5-tris(dodecylaminocarbonyl)cyclohexane, et
- le trans-1,3,5-triméthyl-1,3,5-tris(octadecylaminocarbonyl)cyclohexane.

Les composés de formule I sont bien connus de l'homme de l'art et peuvent être préparés selon les procédés usuels.

Ils sont de préférence présents dans la composition en une quantité aisément déterminable par l'homme du métier en fonction de l'effet recherché, et qui peut être comprise entre 1 et 40% en poids, par exemple 2-10% en poids par rapport au poids total de la composition, et encore mieux 3-8% en poids, voire 4-6% en poids.
On a par ailleurs constaté que même l'utilisation d'une faible quantité de composés de formule I, par exemple de l'ordre de 2-6% en poids, pouvait conduire à une gélification adéquate de la composition selon l'invention. Ceci est dû à un fort pouvoir épaississant des composés de formule I, qui leur permet d'être efficace à faible concentration, de l'ordre de 2-6% en poids, alors qu'il serait nécessaire d'utiliser 10-20% en poids de gélifiants usuels pour obtenir un résultat équivalent.
Sans être tenu par la présente explication, on a constaté que la structuration, ou gélification, des huiles grâce aux composés de formule I pouvait être due à la formation d'amoncellements sous forme de colonnes des molécules de composés de formule I, d'où la constitution d'un réseau de fibres ou feuillets, constitué par lesdits composés de formule I et par les huiles, ledit réseau ne diffractant pas la lumière, d'où une certaine translucidité, voire transparence.

La composition selon l'invention comprend par ailleurs au moins une huile, liquide à température ambiante (25°C) cosmétiquement ou dermatologiquement acceptable.
Ces huiles peuvent être des huiles hydrocarbonées et/ou siliconées et/ou fluorées. Elles peuvent être d'origine animale, végétale, minérale ou synthétique.
On peut en particulier citer :
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque; les huiles de tournesol, de maïs, de soja, de courge, de pépins de raisin, d'arachide, d'amande douce, de calophyllum, de palme, de sésame, de noisette, d'abricot, de macadamia, de ricin, d'avocat; les triglycérides des acides caprylique/caprique comme ceux vendus par la société Stearineries Dubois ou ceux vendus sous les dénominations Miglyol 810, 812 et 818 par la société Dynamit Nobel; l'huile de jojoba, de beurre de karité ;
- les hydrocarbures linéaires ou ramifiés, d'origine minérale ou synthétique tels que les huiles de paraffine et leurs dérivés, la vaseline, les polydécènes, l'huile de Purcellin, le polyisobutène hydrogéné tel que le parléam ;
- les esters et les éthers de synthèse notamment d'acides gras comme les huiles de formule R₃COOR₄ dans laquelle R₃ représente le reste d'un acide gras supérieur comportant de 7 à 29 atomes de carbone et R₄ représenté une chaîne hydrocarbonée contenant de 3 à 30 atomes de carbone comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ;
- des alcools gras ayant de 12 à 26 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées;
- les huiles siliconées comme les polyméthylsiloxanes (PDMS) volatiles ou non, linéaires ou cycliques; les alkyldiméthicones; les silicones modifiées par des groupements aliphatiques et/ou aromatiques, éventuellement fluorés, ou par des groupements fonctionnels tels que des groupements hydroxyles, thiols et/ou amines; les huiles siliconées phénylées telles que les polyphénylméthylsiloxanes ou les phényltriméthicones.
- leurs mélanges.

Les huiles employées peuvent être volatiles et/ou non volatiles. Par huile volatile, on entend une huile susceptible de s'évaporer à température ambiante d'un support sur lequel elle a été appliquée, autrement dit une huile ayant une tension de vapeur mesurable à 25°C et 1 atmosphère, par exemple supérieure à 0 Pa, en particulier allant de 10⁻³ à 300 mm de Hg (0,13 Pa à 40.000 Pa).
On peut notamment citer les huiles siliconées volatiles, telles que les silicones volatiles cycliques ou linéaires, et les cyclocopolymères. On peut également citer les huiles volatiles hydrocarbonées telles que les isoparaffines, et les huiles fluorées volatiles.
Dans un mode de réalisation particulier, les huiles volatiles peuvent constituer la majeure partie de la phase huileuse. Ainsi, elles peuvent y être présentes à raison de au moins 50% en poids, notamment au moins 75% en poids, voire 100% en poids, de ladite phase huileuse.

Les huiles peuvent être présentes dans la composition à raison de 5 à 99% en poids du poids total de la composition, de préférence de 20 à 75% en poids.

La composition selon l'invention se présente sous forme solide. On entend par là qu'on n'observe aucun affaissement de la composition en dehors du récipient la comprenant, en l'absence de stimulation mécanique ou thermique (chauffage notamment).
La composition présente un comportement viscoélastique classique d'un comportement de type solide.

Par ailleurs, la dureté de la composition selon l'invention est de préférence telle que la composition est autoportée et peut se déliter aisément pour former un dépôt satisfaisant sur la peau et les lèvres. Cette dureté peut être comprise entre 0,04 N et 3 N, de préférence entre 0,1 et 2,5 N, notamment entre 0,5 et 2N. Cette dureté peut être mesurée selon une méthode de pénétration d'une sonde dans ladite composition et en particulier à l'aide d'un analyseur de texture (par exemple-TA-XT2 de chez Rhéo) équipé d'un cône en acrylique d'angle au sommet de 45°. La mesure de dureté est effectuée à 22°C au centre de 5 échantillons de ladite composition selon la méthode décrite dans les exemples.

De manière avantageuse, cette composition comprend peu, voire pas, de cire. On entend par là que la composition comprend moins de environ 5% en poids de cire, par rapport au poids total de la composition, de préférence moins de 2% en poids, voire moins de 0,5% en poids de cire. Préférentiellement, la composition ne contient pas de cires (soit 0%).

Une cire, au sens de la présente invention, est un composé gras lipophile, solide à température ambiante (environ 25°C), à changement d'état solide/liquide réversible, ayant une température de fusion supérieure à environ 40°C pouvant aller jusqu'à 200°C, et présentant à l'état solide une organisation cristalline anisotrope.
D'une manière générale, la taille des cristaux de la cire est telle que les cristaux diffractent et/ou diffusent la lumière, conférant à la composition qui les comprend un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

Les cires, au sens de la demande, sont celles généralement utilisées dans les domaines cosmétique et dermatologique.
Elles sont notamment naturelles d'origine animale, végétale ou minérale, comme la cire d'abeilles, la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège.
On peut également citer les cires de paraffine, les cires microcristallines, la cire de lanoline, les ozokérites, les huiles hydrogénées ayant une température de fusion supérieure à environ 40°C comme l'huile de jojoba hydrogénée, les cires de polyéthylène issues de la polymérisation de l'éthylène, les cires obtenues par synthèse de Fischer-Tropsch, les esters d'acide gras et les glycérides ayant une température de fusion supérieure à environ 40°C, les cires de silicone comme les alkyle, alcoxy et/ou esters de poly(di)méthylsiloxane solide à 40°C.

La composition selon l'invention peut comprendre par ailleurs les constituants usuellement utilisés dans le type d'application envisagé.
Elle peut comprendre un ou plusieurs solvants organiques, notamment choisis parmi:
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde.

Il est en outre possible d'incorporer dans la composition selon l'invention une phase hydrophile, notamment en une quantité de 0-10% en poids par rapport au poids total de la composition, et mieux de 1-5% en poids, pouvant comprendre des actifs hydrophiles et/ou des gélifiants hydrophiles. Elle peut notamment comprendre des hydratants tels que la glycérine.

Avantageusement, la composition comprend une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 40% du poids total de la composition, de préférence de 5 à 25% en poids.

Ainsi, la composition peut comprendre une phase particulaire, généralement présente à raison de 0-30% en poids, de préférence 0-20% en poids, et qui peut comprendre des pigments et/ou des nacres et/ou des charges habituellement utilisés dans les compositions cosmétiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.
Les pigments peuvent être blancs ou colorés, minéraux et/ou organiques, de taille micrométrique ou nanométrique. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.
Parmi les nacres envisageables, on peut citer le mica recouvert d'oxyde de titane, d'oxyde de fer, de pigment naturel ou d'oxychlorure de bismuth ainsi que le mica titane coloré.
Les charges peuvent être minérales ou de synthèse, lamellaires ou non lamellaires. On peut citer le talc, le mica, la silice, le kaolin, les poudres de Nylon et de polyéthylène, le Téflon, l'amidon, le micatitane, la nacre naturelle, le nitrure de bore, les microsphères telles que l'Expancel (Nobel Industrie), le polytrap (Dow Corning) et des microbilles de résine de silicone (Tospearls de Toshiba, par exemple).

La composition selon l'invention peut comprendre en outre tout additif usuellement utilisé dans le domaine considéré, notamment le domaine cosmétique, tel que des antioxydants, des parfums, des colorants, des huiles essentielles, des conservateurs, des actifs cosmétiques, des vitamines, des acides gras essentiels, des sphingolipides, des composés auto-bronzants tels que la DHA, des filtres solaires, des tensioactifs, des gélifiants, des polymères notamment hydrocarbonés, tels que le polybutène, les polyalkylènes, les polyacrylates et les polymères ou dérivés siliconés compatibles avec les corps gras. Ces additifs peuvent être présents dans la composition à raison de 0-10% en poids.
Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Les compositions selon l'invention sont destinées à être appliquées sur la peau du visage et du corps, sur les muqueuses et/ou sur les fibres kératiniques telles que les ongles, les cils ou les cheveux.
Elles peuvent se présenter sous toutes les formes galéniques envisageables, telles que gel huileux solide, comprenant éventuellement de l'eau; émulsion solide; système multiphases notamment biphase. Elles peuvent avoir l'aspect d'un solide coulé ou moulé et notamment d'un stick.
Elles peuvent notamment se présenter sous forme de stick ou de coupelle; et en particulier sous forme d'un gel rigide anhydre transparent, et plus spécialement sous forme de stick anhydre transparent.

La gélification de l'huile est telle que l'on peut obtenir une structure rigide sous forme d'un bâton ou d'un stick. Ces bâtons lorsqu'ils sont colorés permettent, après application, d'obtenir un dépôt homogène en couleur et ne migrant pas dans les rides et ridules de la peau, entourant en particulier les lèvres, mais aussi les yeux.

Ces compositions trouvent notamment une application comme composition d'hygiène corporelle, par exemple sous forme de sticks déodorants; comme composition capillaire, par exemple comme stick de coiffage ou stick de maquillage des cheveux; comme composition de maquillage de la peau du visage ou du corps, ou des muqueuses, par exemple comme rouge à lèvres, fond de teint coulé en stick ou en coupelle, fard à joues ou paupières, base fixante à appliquer sur un rouge à lèvres classique, stick anti-cernes, brillant à lèvres, eye-liner, mascara, produits de tatouage éphémère; comme composition de soin de la peau ou des muqueuses.

Ces compositions trouvent une application toute particulière comme composition de maquillage ou de soin non transfert, notamment comme rouge à lèvre non transfert ou fond de teint non transfert.

L'invention est illustrée plus en détail dans les exemples suivants.

### Exemple 1

Le composé utilisé dans cet exemple correspond à la formule I dans laquelle R représente l'hydrogène et Y représente -CO-NHR' avec R' représentant une chaîne alkyle linéaire ayant 18 atomes de carbone.

On mélange sous agitation à température ambiante :
- 250 mg de ce composé
- 5 ml d'isododécane,
soit un mélange à 5% de composé de formule I.

Le mélange est chauffé à 120°C sous agitation, jusqu'à homogénéisation. Il devient alors transparent, homogène et fluide. On laisse alors le mélange homogène refroidir lentement jusqu'à température ambiante (25°C).
On obtient alors une composition solide et dure, qui ne s'affaisse pas en dehors du récipient, en l'absence de toute stimulation mécanique ou thermique. Cette composition peut être étalée par simple pression et permet l'obtention d'un film huileux et homogène.

### Exemple 2

On mélange sous agitation à température ambiante :
- 250 mg du composé de formule I de l'exemple 1
- 5 ml d'isododécane, et
- 25 mg de pigment (oxydes de fer)

Le mélange est chauffé à 120°C jusqu'à homogénéisation. Il devient transparent, coloré, homogène et fluide. On laisse alors le mélange refroidir lentement jusqu'à température ambiante.

On obtient alors une composition solide et colorée, sous la forme d'un stick. Cette composition ne montre pas de séparation du pigment dans le temps. Elle permet l'obtention d'un film huileux et homogène.

### Exemple 3

Le composé de formule I utilisé dans cet exemple correspond à la formule I dans laquelle R représente l'hydrogène et Y représente -CO-NHR' avec R' représentant une chaîne alkyle linéaire ayant 12 atomes de carbone.

On mélange sous agitation à température ambiante :
- 200 mg de ce composé et
- 5 ml d'isododécane,
soit un mélange à 4% en poids de composé de formule I.

Le mélange est chauffé à 120°C sous agitation, jusqu'à homogénéisation. Il devient transparent, homogène et fluide. On laisse alors le mélange homogène refroidir lentement jusqu'à température ambiante.

On obtient alors une composition translucide presque transparente, solide et dure, qui ne s'affaisse pas en dehors du récipient en l'absence de toute stimulation mécanique ou thermique. Elle peut se présenter sous forme de stick. Cette composition permet l'obtention d'un film huileux et homogène.

### A/

On mesure la dureté du stick obtenu, à l'aide d'un analyseur de texture TA-XT2 (société Rhéo), à 22°C, en utilisant un cône lisse en acrylique, d'angle au sommet 45°, et de hauteur totale supérieure à la distance de pénétration. Le cône pénètre à l'intérieur de l'échantillon d'une distance de 5 mm, à une vitesse de 2 mm/s. Il est ensuite maintenu immobile pendant 300 s, puis retiré de l'échantillon à une vitesse de 2 mm/s. La force exercée par l'échantillon sur le corps de mesure est enregistrée en continu.
La force maximale est détectée à la fin de la phase de pénétration. Cette valeur de force reflète la dureté de l'échantillon.

Dans le cas présent, on obtient une dureté de 0,86 N (reproductible).

### B/

La mesure de la transparence ou de la translucidité est effectuée par mesure de la transmittance, soit le pourcentage de lumière transmise à travers un échantillon donné, dans le domaine des longueurs d'onde correspondant au domaine visible, soit entre 400 et 800 nm.

Cette transmittance est mesurée en continu au travers d'un échantillon d'huile épaissie, placé dans une cuve de verre de chemin optique 1 cm, par différence avec un échantillon dit de référence contenant la même huile pure.
L'instrument de mesure est un spectrophotomètre PERKIN-ELMER Lambda UV-Vis.

La composition ci-dessus est chauffée jusqu'à ce qu'elle soit sous forme d'un fluide homogène et est versée directement dans la cuve de mesure. La cuve est maintenue à température ambiante jusqu'au refroidissement de son contenu. On place ensuite la cuve dans l'appareil, la cuve de référence contenant de l'isododécane pur étant placée dans l'appareil également.
On mesure la transmittance entre 400 et 800 nm.
Elle varie de manière continue quasi-linéaire, de 7% à 400 nm à 37% à 800 nm (valeur maximale).

### Exemple 4 (exemple comparatif)

1/ On mélange 200 mg de cire de Carnauba avec 5 ml d'isododécane (mélange à 4% en poids), sous agitation à température ambiante. Le mélange est chauffé à 120°C sous agitation, jusqu'à homogénéisation. On laisse le mélange homogène et fluide refroidir lentement jusqu'à température ambiante.
   Lors du refroidissement, on constate la formation d'un système biphasique mou avec des grains de cire dans une phase huileuse surnageante. Il n'est pas possible d'obtenir une composition solide.
2/ De manière similaire, on prépare un mélange comprenant 500 mg de cire de Carnauba avec 5 ml d'isododécane (mélange à 10% en poids).
   Après refroidissement, on obtient un stick homogène, ayant un dureté de 1,6 N. Toutefois, ce stick est complètement opaque.

On mesure sa transmittance de manière similaire à l'exemple 3; la transmittance est nulle sur toute la longueur du domaine de longueurs d'onde parcouru. Ceci correspond bien à un échantillon totalement opaque

### Exemple 5

De manière similaire aux exemples précédents, on prépare une composition selon l'invention comprenant :
- composé de l'exemple 3 0,8 g
- pigments (oxydes de fer) 0,5 g
- isododécane 16 ml
- huile de parléam 4 ml

On obtient un stick solide dur et coloré.

A l'aide de la composition ainsi préparée, on dépose un film coloré sur une plaque de verre. On laisse le dépôt sécher pendant 20 minutes. Le dépôt est alors sec mais reste malléable.
On applique un mouchoir en papier sur le dépôt, et on presse manuellement. On ne constate aucune trace colorée sur le mouchoir.
Le frottement mécanique du mouchoir sur le dépôt n'entraîne aucun transfert de couleur (entraînement de matière éventuellement).
La composition ainsi préparée présente bien de bonnes propriétés de non-transfert.

## Revendications

1. Composition cosmétique ou dermatologique, se présentant sous forme solide, comprenant au moins une huile et au moins un composé défini par la formule I suivante : 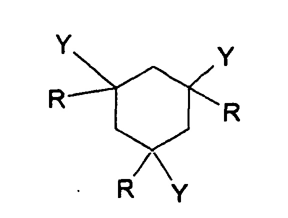 dans laquelle :
* R représente, indépendamment les uns des autres, un atome d'hydrogène ou une chaîne hydrocarbonée saturée, linéaire ou ramifiée, ayant 1 à 6 atomes de carbone, notamment 1 à 4 atomes de carbone;
* Y représente un groupement choisi parmi les groupements suivants : -CO-S-R'; -CO-NHR'; -NH-COR' et -S-COR'; dans lesquels R' représente, indépendamment les uns des autres :
- un atome d'hydrogène,
- un groupement aryle,
- un groupement aralkyle, c'est-à-dire aryle substitué par une chaîne hydrocarbonée saturée, linéaire ou ramifiée, comprenant 1 à 22 atomes de carbone, notamment 10-18 atomes de carbone; ou
- une chaîne hydrocarbonée saturée, linéaire, ramifiée ou cyclique, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy;
ladite composition comprenant moins de environ 5% en poids de cire, par rapport au poids total de la composition.

2. Composition selon la revendication 1, dans laquelle, dans le composé de formule I, on choisit :
- R représente un atome d'hydrogène.
- Y représente un groupement -CO-NHR' ou -NH-COR', et/ou
- R' représente un groupement aryle; un groupement aralkyle dans lequel la chaîne alkyle, linéaire ou ramifiée, comprend 12-16 atomes de carbone; ou une chaîne alkyle linéaire ou ramifiée en C12-18.

3. Composition selon l'une des revendications précédentes, dans laquelle, dans le composé de formule I, R représente un atome d'hydrogène et Y représente un groupement CO-NHR¹ dans lequel R¹ représente un groupement aryle substitué par une chaîne alkyle linéaire ou ramifiée, en C12-C16; ou R' représente une chaîne alkyle linéaire ou ramifiée, en C12-C18, non substituée.

4. Composition selon l'une des revendications précédentes, dans laquelle le composé de formule I est choisi parmi :
- le cis-1,3,5-tris(dodecylaminocarbonyl)cyclohexane,
- le cis-1,3,5-tris(octadecylaminocarbonyl)cyclohexane,
- le cis-1,3,5-tris[N-(3,7-diméthyloctyl)-aminocarbonyl]cyclohexane,
- le cis-1,3,5-tris[N-(3,7-diméthyloctyl)-aminocarbonyl]cyclohexane,
- le trans-1,3,5-triméthyl-1,3,5-tris(dodecylaminocarbonyl)cyclohexane, et
- le trans-1,3,5-triméthyl-1,3,5-tris(octadecylaminocarbonyl)cyclohexane.

5. Composition selon l'une des revendications précédentes, dans laquelle le composé de formule I est présent à raison de 1-40% en poids, par exemple 2-10% en poids par rapport au poids total de la composition, et encore mieux 3-8% en poids, voire 4-6% en poids.

6. Composition selon l'une des revendications précédentes, dans laquelle l'huile est choisie parmi, seules ou en mélange, les huiles hydrocarbonées et/ou siliconées et/ou fluorées, d'origine animale, végétale, minérale ou synthétique.

7. Composition selon l'une des revendications précédentes, dans laquelle l'huile est choisie parmi, seules ou en mélange, :
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque; les huiles de tournesol, de maïs, de soja, de courge, de pépins de raisin, d'arachide, d'amande douce, de calophyllum, de palme, de sésame, de noisette, d'abricot, de macadamia, de ricin, d'avocat; les triglycérides des acides caprylique/caprique comme ceux vendus par la société Stearineries Dubois ou ceux vendus sous les dénominations Miglyol 810, 812 et 818 par la société Dynamit Nobel; l'huile de jojoba, de beurre de karité ;
- les hydrocarbures linéaires ou ramifiés, d'origine minérale ou synthétique tels que les huiles de paraffine et leurs dérivés, la vaseline, les polydécènes, l'huile de Purcellin, le polyisobutène hydrogéné tel que le parléam ;
- les esters et les éthers de synthèse notamment d'acides gras comme les huiles de formule R₃COOR₄ dans laquelle R₃ représente le reste d'un acide gras supérieur comportant de 7 à 29 atomes de carbone et R₄ représente une chaîne hydrocarbonée contenant de 3 à 30 atomes de carbone comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ;
- des alcools gras ayant de 12 à 26 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées;
- les huiles siliconées comme les polyméthylsiloxanes (PDMS) volatiles ou non, linéaires ou cycliques; les alkyldiméthicones; les silicones modifiées par des groupements aliphatiques et/ou aromatiques, éventuellement fluorés, ou par des groupements fonctionnels tels que des groupements hydroxyles, thiols et/ou amines; les huiles siliconées phénylées telles que les polyphénylméthylsiloxanes ou les phényltriméthicones;
- les huiles siliconées volatiles, telles que les silicones volatiles cycliques ou linéaires, et les cyclocopolymères; les huiles volatiles hydrocarbonées telles que les isoparaffines, et les huiles fluorées volatiles.

8. Composition selon l'une des revendications précédentes, dans laquelle les huiles peuvent être présentes à raison de 5 à 99% en poids du poids total de la composition, de préférence de 20 à 75% en poids.

9. Composition selon l'une des revendications précédentes, comprenant moins de environ 2% en poids de cire, par rapport au poids total de la composition, de préférence moins de 0,5% en poids, voire 0% de cire.

10. Composition selon l'une des revendications précédentes, comprenant une matière colorante qui peut être choisie parmi les colorants lipophiles, les colorants hydrophiles, les pigments et les nacres habituellement utilisés dans les compositions cosmétiques ou dermatologiques, et leurs mélanges.

11. Composition selon la revendication 10 dans laquelle la matière colorante est présente à raison de 0,01 à 40% du poids total de la composition, de préférence de 5 à 25% en poids.

12. Composition selon l'une des revendications précédentes, se présentant sous la forme d'une composition d'hygiène corporelle, par exemple sous forme de sticks déodorants; d'une composition capillaire, par exemple comme stick de coiffage ou stick de maquillage des cheveux; d'une composition de maquillage de la peau du visage ou du corps, ou des muqueuses, par exemple comme rouge à lèvres, fond de teint coulé en stick ou en coupelle, fard à joues ou paupières, base fixante à appliquer sur un rouge à lèvres classique, stick anti-cernes, brillant à lèvres, eye-liner, mascara, produits de tatouage éphémère; d'une composition de soin de la peau ou des muqueuses.

13. Composition selon l'une des revendications précédentes, se présentant sous forme translucide voire transparente.

14. Composition selon l'une des revendications précédentes, se présentant sous la forme d'un stick anhydre translucide voire transparent.

15. Composition selon l'une des revendications précédentes, ayant une valeur de transmittance maximum de la lumière, quelque soit la longueur d'onde comprise entre 400 et 800 nm, à travers un échantillon de 1 cm d'épaisseur, d'au moins 2%.

16. Composition selon l'une des revendications précédentes, ayant une dureté comprise entre 0,04 N et 3 N, de préférence entre 0,1 et 2,5 N, notamment entre 0,5 et 2N.

17. Composition selon l'une des revendications précédentes, se présentant sous la forme d'une composition "sans transfert" ou "non migrante" éventuellement colorée.

18. Procédé de traitement cosmétique d'un support choisi parmi la peau du visage ou du corps, les muqueuses et les fibres kératiniques, comprenant l'application sur ledit support d'une composition telle que définie dans l'une des revendications précédentes.

19. Utilisation, dans une composition cosmétique ou dermatologique se présentant sous forme solide, comprenant au moins une huile et comprenant moins de environ 5% en poids de cire par rapport au poids total de la composition, d'une quantité suffisante d'au moins un composé de formule I, pour structurer/gélifier ladite composition.

## Patentansprüche

1. Kosmetische oder dermatologische Zusammensetzung, die in fester Form vorliegt und die mindestens ein Öl und mindestens eine Verbindung der folgenden Formel (I) enthält: 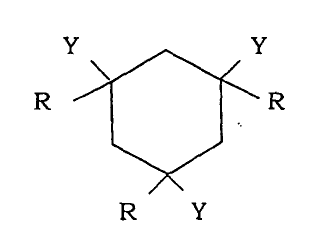 worin:
· die Gruppen R unabhängig voneinander Wasserstoff oder eine geradkettige oder verzweigte, gesättigte Kohlenwasserstoffgruppe mit 1 bis 6 Kohlenstoffatomen und insbesondere 1 bis 4 Kohlenstoffatomen bedeuten; und
· die Gruppen Y unter den folgenden Gruppen ausgewählt sind:
- CO-S-R'; -CO-NHR'; -NH-COR' und -S-COR', wobei die Gruppen R' unabhängig voneinander bedeuten:
- Wasserstoff,
- eine Arylgruppe,
- eine Aralkylgruppe, d.h. eine Arylgruppe, die mit einer gesättigten, geradkettigen oder verzweigten Kohlenwasserstoffgruppe mit 1 bis 22 Kohlenstoffatomen und insbesondere 10 bis 18 Kohlenstoffatomen substituiert ist; oder
- eine gesättigte, geradkettige, verzweigte oder cyclische Kohlenwasserstoffgruppe mit 1 bis 22 Kohlenstoffatomen und insbesondere 10 bis 18 Kohlenstoffatomen, die gegebenenfalls mit einer oder mehreren Gruppen substituiert ist, die unter den Gruppen Aryl, Ester, Amid und Urethan ausgewählt sind; und/oder die gegebenenfalls ein oder mehrere Heteroatome enthält, die unter O, S und N ausgewählt sind; und/oder die gegebenenfalls mit einem oder mehreren Fluoratomen und/oder einer oder mehreren Hydroxygruppen substituiert ist;
wobei die Zusammensetzung weniger als etwa 5 Gew.-% Wachs, bezogen auf das Gesamtgewicht der Zusammensetzung, enthält.

2. Zusammensetzung nach Anspruch 1, wobei für die Verbindung der Formel (I) gewählt werden:
- die Gruppe R bedeutet ein Wasserstoffatom,
- die Gruppe Y ist eine Gruppe -CO-NHR' oder -NH-COR', und/oder
- die Gruppe R' bedeutet eine Arylgruppe; eine Aralkylgruppe, deren geradkettige oder verzweigte Alkylgruppe-12 bis 16 Kohlenstoffatomen enthält; oder eine geradkettige oder verzweigte Alkylgruppe mit 12 bis 18 Kohlenstoffatomen.

3. Zusammensetzung nach einem der vorhergehenden Ansprüche, wobei in der Formel (I) R ein Wasserstoffatom bedeutet und Y die Gruppe -CO-NHR' ist, wobei R' eine mit einer geradkettigen oder verzweigten Alkylgruppe mit 12 bis 16 Kohlenstoffatomen substituierte Arylgruppe oder eine unsubstituierte, geradkettige oder verzweigte Alkylgruppe mit 12 bis 18 Kohlenstoffatomen ist.

4. Zusammensetzung nach einem der vorhergehenden Ansprüche, wobei die Verbindung der Formel (I) ausgewählt ist unter:
- cis-1,3,5-Tris(dodecylaminocarbonyl)cyclohexan,
- cis-1,3,5-Tris(octadecylaminocarbonyl)cyclohexan,
- cis-1,3,5-Tris(N-(3,7-dimethyloctyl)-aminocarbonyl]cyclohexan,
- trans-1,3,5-Trimethyl-1,3,5-tris(dodecylaminocarbonyl)-cyclohexan, und
- trans-1,3,5-Trimethyl-1,3,5-tris(octadecylaminocarbonyl)-cyclohexan.

5. Zusammensetzung nach einem der vorhergehenden Ansprüche, wobei die Verbindung der Formel (I) in einer Menge von 1 bis 40 Gew.-%, beispielsweise 2 bis 10 Gew.-%, bezogen auf das Gesamtgewicht der Zusammensetzung, noch besser 3 bis 8 Gew.-% und sogar 4 bis 6 Gew.-% vorliegt.

6. Zusammensetzung nach einem der vorhergehenden Ansprüche, wobei das Öl unter den Kohlenwasserstoffölen und/oder Siliconölen und/oder fluorierten Ölen tierischen, pflanzlichen, mineralischen oder synthetischen Ursprungs oder deren Gemischen ausgewählt ist.

7. Zusammensetzung nach einem der vorhergehenden Ansprüche, wobei das Öl unter den folgenden Ölen oder deren Gemischen ausgewählt ist:
- Kohlenwasserstoffölen tierischer Herkunft, wie Perhydrosqualen;
- pflanzlichen Kohlenwasserstoffölen, wie beispielsweise flüssigen Triglyceriden von Fettsäuren mit 4 bis 10 Kohlenstoffatomen, beispielsweise Triglyceriden von Heptansäure oder Octansäure; Sonnenblumenöl, Maisöl, Sojaöl, Kürbiskernöl, Traubenkernöl, Erdnussöl, Süßmandelöl, Calophyllumöl, Palmöl, Sesamöl, Haselnussöl, Aprikosenkemöl, Macadamiaöl, Ricinusöl, Avocadoöl; Triglyceriden von Capryl/Caprinsäure, beispielsweise die von der Firma Stearineries Dubois im Handel erhältlichen Produkten oder den Produkten, die unter den Bezeichnungen Miglyol 810, 812 und 818 von der Firma Dynamit Nobel erhältlich sind; Jojobaöl, Sheabutter;
- geradkettigen oder verzweigten Kohlenwasserstoffen mineralischer oder synthetischer Herkunft, beispielsweise Paraffinölen und ihren Derivaten, Vaseline, Polydecenen, Purcellinöl, hydriertem Polyisobuten, wie Parleam;
- synthetischen Estern und Ethern, insbesondere von Fettsäuren, beispielsweise Ölen der Formel R₃COOR₄, in der R₃ den Rest einer höheren Fettsäure mit 7 bis 29 Kohlenstoffatomen bedeutet und R₄ eine Kohlenwasserstoffkette mit 3 bis 30 Kohlenstoffatomen ist, wie beispielsweise Purcellinöl, Isopropylmyristat, 2-Ethylhexylpalmitat, 2-Octyldodecylstearat, 2-Octyldodecylerucat, Isostearylisostearat; hydroxylierte Ester, wie Isostearyllactat, Octylhydroxystearat, Octyldodecylhydroxystearat, Diisostearylmalat, Trüsocetylcitrat, Heptanoaten, Octanoaten und Decanoaten von Fettalkoholen; Estern von Polyolen, wie Propylenglykoldioctanoat, Neopentylglykoldiheptanoat, Diethylenglykoldiisononanoat; und den Estern von Pentaerythrit;
- Fettalkoholen mit 12 bis 26 Kohlenstoffatomen, wie Octyldodecanol, 2-Butyloctanol, 2-Hexyldecanol, 2-Undecylpentadecanol, Oleylalkohol;
- fluorierten Ölen, die teilweise kohlenwasserstoffhaltig und/oder siliconhaltig sind;
- Siliconölen, wie Polydimethylsiloxanen (PDMS), die flüchtig oder nicht flüchtig, geradkettig oder cyclisch vorliegen können; Alkyldimethiconen; mit aliphatischen und/oder aromatischen, gegebenenfalls fluorierten Gruppen oder mit funktionellen Gruppen modifizierten Siliconen, beispielsweise mit Hydroxygruppen, Thiolgruppen und/oder Aminogruppen modifizierten Siliconen; phenylierten Siliconölen, wie Polyphenylmethylsiloxanen oder Phenyltrimethiconen; und
- flüchtigen Siliconölen, beispielsweise cyclischen oder geradkettigen flüchtigen Siliconen und Cyclocopolymeren; flüchtigen Kohlenwasserstoffölen, wie Isoparaffinen, und flüchtigen fluorierten Ölen.

8. Zusammensetzung nach einem der vorhergehenden Ansprüche, wobei die Öle in einer Menge von 5 bis 99 Gew.-%, des Gesamtgewichts der Zusammensetzung und vorzugsweise 20 bis 75 Gew.-% enthalten sein können.

9. Zusammensetzung nach einem der vorhergehenden Ansprüche, die weniger als etwa 2 Gew.-% Wachs, bezogen auf das Gesamtgewicht der Zusammensetzung, vorzugsweise weniger als 0,5 Gew.-% Wachs und sogar 0 % Wachse enthält.

10. Zusammensetzung nach einem der vorhergehenden Ansprüche, die ein Farbmittel enthält, das unter den lipophilen Farbmitteln, hydrophilen Farbmitteln, Pigmenten oder Perlglanzpigmenten, die üblicherweise in kosmetischen oder dermatologischen Zusammensetzungen verwendet werden, und deren Gemischen ausgewählt ist.

11. Zusammensetzung nach Anspruch 10, wobei das Farbmittel in einer Menge von 0,01 bis 40 % des Gesamtgewichts der Zusammensetzung und vorzugsweise in einer Menge von 5 bis 25 % enthalten ist.

12. Zusammensetzung nach einem der vorhergehenden Ansprüche, die in Form einer Zusammensetzung zur Körperpflege, beispielsweise als Deostift; als Zusammensetzung für die Haarbehandlung, beispielsweise als Stift für die Frisurengestaltung oder zum Schminken der Haare; als Zusammensetzung zum Schminken der Haut des Gesichts oder des Körpers oder der Schleimhäute, beispielsweise als Lippenstift, Make-up in Stiftform oder in Tiegelchen gegossen, Wangenrouge, Lidschatten, fixierende Grundmasse, die über einem herkömmlichen Lippenstift aufgetragen wird, Stift gegen Augenringe, Lipgloss, Eyeliner, Mascara, nicht permanentes Produkt zur Tätowierung; Zusammensetzung zur Pflege der Haut oder der Schleimhäute vorliegt.

13. Zusammensetzung nach einem der vorhergehenden Ansprüche, die in durchscheinender oder sogar transparenter Form vorliegt.

14. Zusammensetzung nach einem der vorhergehenden Ansprüche, die als wasserfreier, durchscheinender oder sogar transparenter Stift vorliegt.

15. Zusammensetzung nach einem der vorhergehenden Ansprüche, die durch eine Probe von 1 cm Dicke hindurch im Bereich von 400 bis 800 nm unabhängig von der Wellenlänge einen Wert der maximalen Lichtdurchlässigkeit von mindestens 2 % aufweist.

16. Zusammensetzung nach einem der vorhergehenden Ansprüche, die eine Härte im Bereich von 0,04 bis 3 N, vorzugsweise 0, 1 bis 2,5 N und insbesondere 0,5 bis 2 N aufweist.

17. Zusammensetzung nach einem der vorhergehenden Ansprüche, die in Form einer Zusammensetzung "ohne Transfer" oder "ohne Migration", die gegebenenfalls farbig ist, vorliegt.

18. Verfahren zur kosmetischen Behandlung eines Trägers, der unter der Haut des Gesichts oder des Körpers, den Schleimhäuten und den Keratinsubstanzen ausgewählt ist, das umfasst, auf den Träger eine Zusammensetzung nach einem der vorhergehenden Ansprüche aufzubringen.

19. Verwendung mindestens einer Verbindung der Formel (I) in einer kosmetischen oder dermatologischen Zusammensetzung, die in fester Form vorliegt und mindestens ein Öl und weniger als etwa 5 Gew.-% Wachs, bezogen auf das Gesamtgewicht der Zusammensetzung, in einer ausreichenden Menge enthält, um die Zusammensetzung zu strukturieren/zu gelieren.

## Claims

1. Cosmetic or dermatological composition, which is in solid form, comprising at least one oil and at least one compound defined by formula I below: 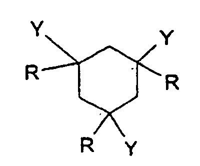 in which:
* R represents, independently of each other, a hydrogen atom or a linear or branched, saturated hydrocarbon-based chain containing 1 to 6 carbon atoms, in particular 1 to 4 carbon atoms;
* Y represents a group chosen from the following groups: -CO-S-R'; -CO-NHR'; -NH-COR' and -S-COR'; in which R' represents, independently of each other:
- a hydrogen atom,
- an aryl group,
- an aralkyl group, i.e. an aryl group substituted with a linear or branched, saturated hydrocarbon-based chain containing 1 to 22 carbon atoms, in particular 10-18 carbon atoms; or
- a linear, branched or cyclic, saturated hydrocarbon-based chain containing 1 to 22 carbon atoms, in particular 10-18 carbon atoms, optionally substituted with one or more groups chosen from aryl, ester, amide and urethane groups; and/or optionally comprising one or more hetero atoms chosen from O, S and N; and/or optionally substituted with one or more fluorine atoms and/or hydroxyl radicals;
the said composition comprising less than about 5% by weight of wax relative to the total weight of the composition.

2. Composition according to Claim 1, in which, in the compound of formula I, the following are chosen:
- R represents a hydrogen atom,
- Y represents a group -CO-NHR' or -NH-COR', and/or
- R' represents an aryl group; an aralkyl group in which the linear or branched alkyl chain contains 12-16 carbon atoms; or a linear or branched C₁₂-C₁₈ alkyl chain.

3. Composition according to either of the preceding claims, in which, in the compound of formula I, R represents a hydrogen atom and Y represents a group -CO-NHR' in which R' represents an aryl group substituted with a linear or branched C₁₂-C₁₆ alkyl chain; or R' represents an unsubstituted, linear or branched C₁₂-C₁₈ alkyl chain.

4. Composition according to one of the preceding claims, in which the compound of formula I is chosen from:
- cis-1,3,5-tris(dodecylaminocarbonyl)cyclohexane,
- cis-1,3,5-tris(octadecylaminocarbonyl)cyclohexane,
- cis-1,3,5-tris(N-(3,7-dimethyloctyl)aminocarbonyl]-cyclohexane,
- trans-1,3,5-trimethyl-1,3,5-tris(dodecylaminocarbonyl)cyclohexane and
- trans-1,3,5-trimethyl-1,3,5-tris(octadecylaminocarbonyl)cyclohexane.

5. Composition according to one of the preceding claims, in which the compound of formula I is present in a proportion of 1-40% by weight, for example 2-10% by weight, relative to the total weight of the composition, and better still 3-8% by weight, or even 4-6% by weight.

6. Composition according to one of the preceding claims, in which the oil is chosen, alone or as a mixture, from hydrocarbon-based oils and/or silicone oils and/or fluoro oils of animal, plant, mineral or synthetic origin.

7. Composition according to one of the preceding claims, in which the oil is chosen, alone or as a mixture, from:
- hydrocarbon-based oils of animal origin such as perhydrosqualene;
- hydrocarbon-based plant oils such as liquid triglycerides of fatty acids of 4 to 10 carbon atoms, such as heptanoic or octanoic acid triglycerides; sunflower oil, corn oil, soybean oil, marrow oil, grapeseed oil, groundnut oil, sweet almond oil, beauty-leaf oil, palm oil, sesame oil, hazelnut oil, apricot oil, macadamia oil, castor oil, avocado oil; caprylic/capric acid triglycerides such as those sold by the company Stearineries Dubois or those sold under the names Miglyol 810, 812 and 818 by the company Dynamit Nobel; jojoba oil, karite butter;
- linear or branched hydrocarbons of mineral or synthetic origin, such as liquid paraffins and derivatives thereof, petroleum jelly, polydecenes, purcellin oil, and hydrogenated polyisobutene such as parleam;
- synthetic esters and ethers, in particular of fatty acids, such as the oils of formula R₃COOR₄ in which R₃ represents a higher fatty acid residue containing from 7 to 29 carbon atoms and R₄ represents a hydrocarbon-based chain containing from 3 to 30 carbon atoms, such as, for example, purcellin oil, isopropyl myristate, 2-ethylhexyl palmitate, 2-octyldodecyl stearate, 2-octyldodecyl erucate, isostearyl isostearate; hydroxylated esters such as isostearyl lactate, octyl hydroxystearate, octyldodecyl hydroxystearate, diisostearyl malate, triisocetyl citrate, and fatty alcohol heptanoates, octanoates and decanoates; polyol esters such as propylene glycol dioctanoate, neopentyl glycol diheptanoate or diethylene glycol diisononanoate; and pentaerythritol esters;
- fatty alcohols containing from 12 to 26 carbon atoms, such as octyldodecanol, 2-butyloctanol, 2-hexyldecanol, 2-undecylpentadecanol or oleyl alcohol;
- partially hydrocarbon-based and/or silicone-containing fluoro oils;
- silicone oils such as volatile or non-volatile, linear or cyclic polydimethylsiloxanes (PDMSs); alkyldimethicones; silicones modified with aliphatic and/or aromatic groups, which are optionally fluorinated, or with functional groups such as hydroxyl, thiol and/or amine groups; phenylsilicone oils such as polyphenylmethylsiloxanes or phenyltrimethicones;
- volatile silicone oils, such as cyclic or linear volatile silicones, and cyclocopolymers; hydrocarbon-based volatile oils such as isoparaffins, and volatile fluoro oils.

8. Composition according to one of the preceding claims, in which the oils can be present in a proportion of from 5% to 99% by weight relative to the total weight of the composition, preferably from 20% to 75% by weight.

9. Composition according to one of the preceding claims, comprising less than about 2% by weight of wax, relative to the total weight of the composition, preferably less than 0.5% by weight, or even 0% of wax.

10. Composition according to one of the preceding claims, comprising a dyestuff which can be chosen from the lipophilic dyes, hydrophilic dyes, pigments and nacres usually used in cosmetic or dermatological compositions, and mixtures thereof.

11. Composition according to Claim 10, in which the dyestuff is present in a proportion of from 0.01% to 40% relative to the total weight of the composition, preferably from 5% to 25% by weight.

12. Composition according to one of the preceding claims, which is in the form of a body hygiene composition, for example in the form of deodorant sticks; a hair composition, for example as styling sticks or make-up sticks for the hair; a make-up composition for facial or body skin or for mucous membranes, for example as lipsticks, foundations cast as a stick or a dish, face powders, eyeshadows, fixing bases to be applied to a conventional lipstick, concealer sticks, lip glosses, eyeliners, mascaras or temporary tattoo products; or a care composition for the skin or mucous membranes.

13. Composition according to one of the preceding claims, which is in translucent or even transparent form.

14. Composition according to one of the preceding claims, which is in the form of a translucent or even transparent anhydrous stick.

15. Composition according to one of the preceding claims, which has a maximum light transmittance value, irrespective of the wavelength between 400 nm and 800 nm, through a 1 cm-thick sample, of at least 2%.

16. Composition according to one of the preceding claims, which has a hardness of between 0.04 N and 3 N, preferably between 0.1 N and 2.5 N, especially between 0.5 N and 2 N.

17. Composition according to one of the preceding claims, which is in the form of an optionally coloured "transfer-resistant" or "non-migrating" composition.

18. Cosmetic treatment process for a support chosen from facial or body skin, mucous membranes and keratin fibres, comprising the application to the said support of a composition as defined in one of the preceding claims.

19. Use, in a cosmetic or dermatological composition, which is in solid form, comprising at least one oil and comprising less than about 5% by weight of wax relative to the total weight of the composition, of a sufficient amount of at least one compound of formula I, to structure/gel the said composition.
